# EUROPÄISCHE PATENTSCHRIFT

(11) **EP 3 149 146 B1**
(45) Veröffentlichungstag und Bekanntmachung des Hinweises auf die Patenterteilung: **29.08.2018**
(21) Anmeldenummer: 15744461.3
(22) Anmeldetag: 27.05.2015
(51) Int. Cl.: C12M 1/00, C12M 1/12, C12M 1/02

(54) **VERFAHREN FÜR EINEN PHOTOCHEMISCHEN, WIE PHOTOKATALYTISCHEN UND/ODER PHOTOSYNTHETISCHEN PROZESS**
METHOD FOR A PHOTOCHEMICAL PROCESS, SUCH AS A PHOTOCATALYTIC AND/OR PHOTOSYNTHETIC PROCESS
PROCÉDÉ POUR UN PROCESSUS PHOTOCHIMIQUE TEL QU'UN PROCESSUS PHOTOCATALYTIQUE ET/OU PHOTOSYNTHÉTIQUE

(30) Priorität: 30.05.2014 AT 4312014
(43) Veröffentlichungstag der Anmeldung: 05.04.2017
(73) Patentinhaber: ecoduna AG, 2460 Bruck an der Leitha (AT)
(72) Erfinder: EMMINGER, Franz, A-2410 Hainburg (AT)
(74) Vertreter: Sonn & Partner Patentanwälte
(86) Internationale Anmeldenummer: PCT/AT2015/000081
(87) Internationale Veröffentlichungsnummer: WO 2015/179888

(56) Entgegenhaltungen:
- WO-A2-2009/051478
- US-A1- 2011 027 875
- US-A1- 2012 003 734

## Beschreibung

Die Erfindung betrifft ein Verfahren für einen photochemischen, wie photokatalytischen und/oder photosynthetischen Prozess, insbesondere für eine Zucht und Produktion bzw. Hydrokultivierung, von, vorzugsweise phototrophen, Mikroorganismen, wobei ein Reaktionsmedium, beispielsweise eine wässerige Lösung oder eine Suspension aus Mikroorganismen und Nährlösung, mäanderförmig in einem Reaktorelement, welches mindestens aus zwei aufrechten, verbundenen Röhren oder Kammern gebildet wird, geführt wird, wobei die mäanderförmige Führung des Reaktionsmediums senkrecht oder gegebenenfalls in einem Winkel zur Sonne geneigt, erfolgt und vorzugsweise mehrere Reaktorelemente seriell zu einem Biosolarreaktor verbunden werden und unter Ausnutzung des hydrostatischen Druck- und Niveauausgleich im Biosolarreaktor eine für die Mikroorganismen stressfreie Strömung des Reaktionsmediums erzeugt wird. Ferner betrifft die Erfindung eine Einrichtung zur Durchführung des Verfahrens sowie einen Biosolarreaktor.

Aus der AT 506 373 B1 ist eine System bekannt, bei dem der Biosolarreaktor aus mindestens einen Reaktorelement besteht, wobei das Reaktorelement aus mindestens zwei aufrechten, unten verbundenen Röhren gebildet ist, um einen mäanderförmigen Verlauf eines Reaktionsmediums zu erreichen. Dabei weist das Reaktionselement einen Einlass und einen Auslass am oberen Reaktorrand auf. Die mäanderförmige Führung des Medium erfolgt senkrecht, wobei aufgrund des hydrostatischen Druck- und Niveaugleiches eine für das Medium stressfreie Strömung erzeugt wird.

Nachteilig ist hierbei, dass bei einer derartigen Anlage das Einbringen und Ausbringen des Mediums auf der Oberseite der Anlage erfolgt, so dass die Zugänglichkeit wesentlich erschwert wird. Insbesondere für die Entnahme von Proben zum Feststellen des Wachstums der Mikroorganismen muss bei derartigen Anlagen dies auf der Oberseite erfolgen.

Eine weitere ähnliche Anlage ist aus der AT 507 989 B1 sowie der US 2012/0003734 A1 bekannt, bei der nunmehr das Reaktorelement in einer vorzugsweise lichtdurchlässigen Flüssigkeit angeordnet ist.

Aus der DE 41 34 813 A1 ist ein Bioreaktor für phototropische Mikroorganismen, welcher aus Glas oder Kunststoff besteht, bekannt. Das Kulturmedium wird entweder durch den Bioreaktor gepumpt oder mäanderförmig durch die waagrecht angeordneten Stegplatten nach unten geleitet. Weiters sind Turbulenz erzeugende Mittel in den Stegen angebracht. Entsprechend diesem Verfahren wird Kohlenstoffdioxid oben eingeleitet und zum Betrieb wird natürliches oder Kunstlicht verwendet. Der Bioreaktor wird in rechtem Winkel zur Lichtquelle gestellt bzw. nachgeführt.

Weiters sind auch aus der GB 2 235 210 A und der DE 196 44 992 C1 Bioreaktoren für phototrope Mikroorganismen bzw. für photokatalytische Prozesse bekannt.

Aus der EP 738 686 A1 ist die photokatalytische Abwasserreinigung in einem Bioreaktor, wo die zu reinigende Flüssigkeit durch Stegmehrfachplatten aus transparentem Kunststoff geleitet wird, bekannt. Für die Regulierung der Temperatur können transluzente handelsübliche Mehrfachstegplatten Verwendung finden.

Ferner ist in der WO 98/18903 ein aktiv oder passiv temperierbares Solarelement aus Mehrfachstegplatten mit mindestens drei Gurten beschrieben. Schichten innerhalb des Reaktors werden wechselweise für einen photochemischen bzw. photosynthetischen Prozess genutzt. Dabei wird in einem geschlossenen Reaktor mit abgedichteter Stirnseite und waagrecht angeordneten Stegplatten das Kulturmedium mäanderförmig nach unten geleitet.

Darüber hinaus ist aus der DE 195 07 149 C2 eine Wasserkraftschnecke mit einem Trog und einem Generator zur Stromgewinnung bekannt. Aus der DE 41 39 134 C2 ist eine Wasserkraftschnecke zur Energieumwandlung | bekannt. WO2009/051478 zeigt eine Anlage, bei der das Reaktionsmedium mäanderförmig durch zumindest teilweise transparente Rohre geführt wird. Die Förderung des Reaktionsmediums erfolgt dabei über eine Pumpe.

Natürlich ist der hydrostatische Kräfteausgleich als hydrostatisches Paradoxon, auch genannt Pascalsches Paradoxon, bekannt. Dieser ist ein scheinbares Paradoxon, welches das Phänomen beschreibt, dass eine Flüssigkeit einen Schweredruck, abhängig von der Füllhöhe der Flüssigkeit auf den Boden eines Gefäßes, bewirkt, die Form des Gefäßes aber keinen Einfluss ausübt.
Als kommunizierende Gefäße oder kommunizierende Röhren benennt man oben offene, unten verbundene Gefäße. Ein homogenes Fluid steht in ihnen in gleicher Höhe, weil der Luftdruck und die Schwerkraft auf die Gefäße gleichermaßen wirken. Bei inhomogenen Flüssigkeiten verhalten sich die Flüssigkeitssäulen in der Höhe umgekehrt zu ihrem spezifischen Gewicht.
Üblicherweise wird - wie auch in einigen oben angeführten Verfahren - der Transport in Solarreaktoren durch handelsübliche Pumpverfahren durchgeführt. Diese Vorgehensweise verursacht Stress im Reaktionsmedium, sei es durch hohen Druck, Unterdruck, starke Beschleunigung oder Quetschung. Diesem Stress ausgesetzt, fallen die meisten phototropischen Mikroorganismen in ihren potenziellen photosynthetischen Fähigkeiten ab. Zellen werden zerstört, geschädigt und/oder die Mikroorganismen brauchen Zeit und/oder Stoffwechselprodukte zur Regeneration, bevor sie die ihnen zugewiesenen Prozesse wieder voll aufnehmen können. Ebenso fallen, diesem Stress ausgesetzt, die meisten photochemischen Prozesse in ihren potenziellen photokatalytischen Fähigkeiten ab, da Moleküle zerstört oder geschädigt werden und/oder Zeit und/oder weitere Oxidationsmittel benötigen, bevor sie die Ihnen zugewiesenen Prozesse wieder voll aufnehmen können.
Aufgabe der Erfindung ist es, ein Verfahren der eingangs zitierten Art zu schaffen, das einerseits die obigen Nachteile vermeidet und das anderseits einen sicheren, wirtschaftlich rationellen Betrieb der Anlage gewährleistet. Eine weitere Aufgabe der Erfindung liegt darin, in der Anlage eine Prozessoptimierung im industriellen Betrieb zu erreichen.
Das erfindungsgemäße Verfahren für einen photochemischen, wie photokatalytischen und/oder photosynthetischen Prozess, insbesondere für eine Zucht und Produktion bzw. Hydrokultivierung von, vorzugsweise phototrophen, Mikroorganismen ist dadurch gekennzeichnet, dass an die einzelnen Reaktorelemente an der Unterseite an jeweils den äußersten Röhren oder Kammern Ein- und Auslassöffnungen angeordnet werden, wobei der Einlassöffnung ein, gegen die Richtung der Schwerkraft, aufsteigender Ast und der Auslassöffnung ein, in Richtung der Schwerkraft, abfallender Ast, zugeordnet wird und dass alle Anschlüsse im unteren Bereich, d.h. die Einlassöffnung, die Auslassöffnung und der Einbringungseinlass, mit dem Reaktionsmedium umspült werden.
Vorteilhaft ist hierbei, dass alle Verbindungen, insbesondere Einlass und Auslass, an der Unterseite des Moduls angeordnet sind, so dass ein einfacheres Handling bei Problemen mit den Verbindungen möglich ist. Beim Stand der Technik sind die Anschlüsse ausnahmslos auf der Oberseite angeordnet. Damit muss man bei der erfindungsgemäßen Konfiguration der Anlage nicht mehr beispielsweise 6 Meter in die Höhe fahren, um Wartungen, Probeentnahmen oder Überprüfungstätigkeiten durchführen zu können.
Ein wesentlicher Vorteil liegt jedoch darin, dass die Verbindungsstücke, insbesondere die Einlassöffnung und die Auslassöffnung, immer mit Flüssigkeit überspült sind, so dass der Betrieb der Anlage wesentlich verbessert wurde, da keine Luft, die sich schädlich auf die Mikroorganismen auswirken kann, eindringen kann. Somit ist auch sichergestellt, dass die für die Verbindungsstücke verwendeten Dichtungen immer mit Flüssigkeit umspült sind und diese damit nicht austrocknen können.

Darüber hinaus sind die Reaktorelemente derart konzipiert, dass beim Zusammenschalten zweier oder mehrere Reaktorelemente keine Pumpen, Motoren o. dgl. benötigt werden, so dass ein schnelles Wachstum der Mikroorganismen im stressfreien Fließen des Reaktionsmediums erzielt wird.

Mit der Erfindung ist es also erstmals möglich, einen für die Mikroorganismen schonenden Transport zu erreichen, so dass eine Schädigung im Zuge ihres Produktionsverfahrens vermieden wird. Das Reaktionsmedium fließt mäanderartig durch die aufrechten miteinander verbundenen Reaktorelemente. Die Reaktorelemente sind so miteinander verbunden, das der Einlass und der Auslass nunmehr auf der Unterseite leicht zugänglich angeordnet sind. Der Durchfluss wird unter Ausnutzung des hydrostatischen Druckausgleiches mit minimalem Höhenverlust innerhalb des gesamten Reaktors erreicht. Durch den weitestgehend druckfreien und appressionsfreien Transport des Reaktionsmediums in einem Biosolarreaktor wird der Reaktionsprozess möglichst wenig beeinträchtigt.

Das erfindungsgemäße Verfahren kann beispielsweise für folgende Anwendungsbereiche eingesetzt werden:
- Die photokatalytische Reinigung von Abwasser
- Die photosynthetische Verstoffwechslung von CO₂ zu Sauerstoff, durch phototropische Mikroorganismen
- Zucht und Produktion von phototropischen Mikroorganismen für Forschungszwecke
- Forschung an photochemischen und oder photosynthetischen Prozessen
- Zucht und Produktion von phototropischen Mikroorganismen für Nahrungsmittelprodukte und Nahrungsmittelgrundstoffe
- Zucht und Produktion von phototropischen Mikroorganismen für Grundstoffe der pharmazeutischen Industrie
- Zucht und Produktion von phototropischen Mikroorganismen für Kraftstoffe und Grundstoffe für Kraftstoffproduktion und Energiegewinnung
- Zucht und Produktion von phototropischen Mikroorganismen für Grundstoffe der chemischen Industrie
- Zucht und Produktion von phototropischen Mikroorganismen die nutzbare Gase, wie beispielsweise Wasserstoff, im photosynthetischen Prozess abgeben

Durch die Nutzung des hydrostatischen Kräfteausgleichs beim Durchfließen des Reaktionsmediums der Reaktorelemente erfolgt quasi ein stressfreier Transport der gegebenenfalls mitgeführten Mikroorganismen. Ferner kann eine Energieoptimierung, eine definierte Lichtführung, eine Platzoptimierung, eine Versorgung mit Zusatzstoffen, eine definierte Temperierung, eine gezielte Steuerung sowie eine verbesserte Gasausbringung erzielt werden.

Gemäß einem weiteren besonderen Merkmal der Erfindung wird eine, kontinuierliche oder chargenweise, Einbringung von flüssigen und/oder gasförmigen Zusatzstoffen, wie insbesondere Nährstofflösungen und/oder Oxidationsmittel und/oder Wirksubstanzen und/oder den Prozess fördernde gelöste Stoffe, vorzugsweise während des Prozesses, an der Unterseite, im Bereich der Einlass- und/oder Auslassöffnungen des Reaktionsmediums, durchgeführt, wobei durch die Einbringung der Zusatzstoffe am unteren Ende der Flüssigkeitssäule eine Durchmischung und gleichmäßige Verteilung der Zusatzstoffe im Reaktionsmedium erfolgt. Dadurch können eine kontrollierte und optimierte Einbringung von Nährlösungen und prozessfördernden Lösungen sowie eine kontrollierte und optimierte Einbringung von Nähr- und Prozessgasen erfolgen. Alle Interventionen in das Reaktionsmedium werden an der Unterseite der Reaktionselemente vorgenommen.

Nach einem weiteren Merkmal der Erfindung wird über eine, vorzugsweise an der Oberseite des Reaktorelementes vorgesehene, Füllstandskontrolle der Füllstand des Reaktionsmediums erfasst und bei Absinken durch Zugabe einer Flüssigkeit und/oder des Reaktionsmediums auf einem voreinstellbaren Wert gehalten. Dadurch wird ein optimaler Prozess, entsprechend den oben aufgezeigten Visionen gewährleistet.

Gemäß einem weiteren besonderen Merkmal der Erfindung erfolgt die Ausbringung von gasförmigen Prozessprodukten, wie beispielsweise Sauerstoff oder CO₂, vorzugsweise während des Prozesses, über die Reaktionsmediums-Oberfläche und wird vorzugsweise über ein Abluftsystem an der Oberseite des Reaktorelementes abtransportiert bzw. abgesaugt. Dadurch kann eine kontrollierte und optimierte Reduktion von Schadstoffen erreicht werden, wobei diese optimierte Ausbringung auch ein Sammeln von gasförmigen Prozessprodukten erlaubt. Durch das Absaugen der Gase wird ein sicherer Betrieb der Anlage erreicht, da keinerlei gifte Gase im Bereich der Anlage auftreten können.
Nach einer weiteren Ausgestaltung der Erfindung wird das Reaktorelement auf der Oberseite durch ein, vorzugsweise abnehmbares, Abschlusselement, verschlossen. Dadurch wird ein Überlaufen des Reaktorelementes verhindert. Gleichzeitig wird verhindert, dass Gase aus der Anlage, insbesondere den Reaktorelementen, austreten können.
Weiters ist es auch Aufgabe der Erfindung eine Einrichtung zur Durchführung des Verfahrens zu schaffen. Insbesondere ist es Aufgabe der Erfindung die Zugänglichkeit der Anlage im industriellen Betrieb zu verbessern.
Die erfindungsgemäße Einrichtung zur Durchführung des Verfahrens, bei der ein Reaktor, insbesondere ein Biosolarreaktor, der aus mindestens einem Reaktorelement besteht und das aus mindestens zwei aufrechten, verbundenen Röhren oder Kammern gebildet ist, ist dadurch gekennzeichnet, dass die einzelnen Reaktorelemente an der Unterseite an jeweils den äußersten Röhren oder Kammern Ein- und Auslassöffnungen aufweisen, wobei der Einlassöffnung ein, gegen die Richtung der Schwerkraft, aufsteigender Ast und der Auslassöffnung ein, in Richtung der Schwerkraft, abfallender Ast zugeordnet ist, dass alle Anschlüsse im unteren Bereich, d.h. die Einlassöffnung, die Auslassöffnung und der Einbringungseinlass, mit dem Reaktionsmedium umspült sind und dass vorzugsweise die Einlass- und/oder Auslassöffnung an der tiefsten Stelle des Reaktorelementes angeordnet ist.

Eine derartige erfindungsgemäße Einrichtung hat den Vorteil, dass der Aufbau an die den jeweiligen phototrophischen Mikroorganismen oder photochemischen Anforderungen angepasste und dem Prozessergebnis entsprechend optimale Verweildauer innerhalb des gesamten Reaktors durch folgende Parameter beeinflusst werden kann:
- Durchflussgeschwindigkeit
- Querschnitt der Reaktorelemente
- Höhe der Reaktorelemente
- Anzahl und Beschaffenheit der eingebrachten, nicht gasförmigen Stoffe
- Beschaffenheit, Anzahl, Dichte und Druck der eingeblasenen Gase
- Die Anzahl der in mäanderartiger Führung verbundenen Reaktorelemente
- Die Möglichkeit Prozessabgase auszubringen
- Prozesstemperaturen
- Verweildauer und Stellung zu Licht
- Verweildauer in Reifungstanks und oder Dunkeltanks

Nach einer alternativen Ausgestaltung der Erfindung ist das Reaktorelement aus mehreren, insbesondere siebzig, vorzugsweise rechteckigen, aufrechten, Kammern bzw. Röhren gebildet, die durch eine Trennwand voneinander getrennt sind, wobei die Trennwand einmal auf der Oberseite und anschließend an der Unterseite, also im Bereich eines Boden, für den Umlauf des Reaktionsmedium offen ist. Der Vorteil liegt darin, dass dadurch die Stabilität der Reaktorelemente erhöht wird, da sich die einzelnen Kammern ohne Zwischenraum aneinander fügen.

Der Reaktor, insbesondere der Bioreaktor kann aus transparenten, transluzenten, beschichteten und unbeschichteten Materialien bestehen. Ebenso könnten die Röhren oder Stegplatten aus Glas oder Licht bzw. UV-Licht durchlässigem Kunststoff wie beispielsweise Polymethylmethacrylat bestehen. Die Reaktorelemente können sowohl aus handelsüblichen und gegebenenfalls bearbeiteten, als auch gesondert gefertigten Bauteilen, die obigen Bedingungen erfüllen, ausgeführt sein.

Die Reaktorelemente werden so angeordnet, dass ein kontinuierliches, mäanderartiges Durchfließen von unten nach oben und von oben nach unten gewährleistet ist. Der Zufluss und der Abfluss zum bzw. aus dem Reaktor ist im unteren Bereich angesetzt.

Nach Eintritt in den Reaktor fließt das Reaktionsmedium, unter Ausnutzung des hydrostatischen Kräfteausgleichs stressfrei, den gesamten Reaktor in aufrechten Mäandern ab. Im letzten Reaktorelement angekommen, verlässt das Reaktionsmedium den hydrostatischen Bioreaktor und wird druckfrei bzw. drucklos zu einem Reifungstank oder einem Auffangbehälter oder einem weiteren Reaktor geführt. Vom Auffangbehälter kann das Reaktionsmedium endbearbeitet oder stressfrei einer Zwischenlagerung oder weiteren Bearbeitung zugeführt werden.

Dadurch, dass die Einlass- und/oder Auslassöffnung an der tiefsten Stelle des Reaktorelementes angeordnet ist, ist ein gleichmäßiges Durchströmen des Reaktors mit der Reaktorflüssigkeit sichergestellt.

Gemäß einem besonderen Merkmal der Erfindung ist bei einer Verbindung von zwei oder mehr Reaktorelementen immer eine Auslassöffnung mit einer Einlassöffnung, vorzugsweise über eine Verbindungsleitung, verbunden. Dadurch ist die Anlage beliebig erweiterbar.

Nach einem weiteren besonderen Merkmal der Erfindung ist für die, kontinuierliche oder chargenweise, Einbringung von Zusatzstoffen, wie beispielsweise Nährstofflösungen bzw. -gasen und/oder Oxidationsmittel und/oder Wirksubstanzen und/oder den Prozess fördernde gelöste Stoffe bzw. Gase, vorzugsweise während des Prozesses, an der Reaktorunterseite, im Bereich der Umlenkung des Reaktionsmediums und/oder über die Verbindungsleitung, mindestens ein Einbringungseinlass vorgesehen. Das Reaktionsmedium kann wahlweise vor dem Eintritt in den Reaktor mit in Flüssigkeiten gelösten Stoffen die den Bedürfnisse der Mikroorganismen oder Anforderungen des Prozesses entsprechen, angereichert, und/oder während des Durchlaufes im Reaktor mit fluiden Nährstoffen oder Oxidationsmitteln versorgt werden.

Der im photosynthetischen Prozess, durch stetes Heranwachsen der Mikroorganismen, sinkende Nährstoffgehalt im Reaktionsmedium kann durch die kontinuierliche und/oder getagte Einbringung einer Nährstofflösung ausgeglichen werden.

Der im photochemischen Prozess durch stetes Reagieren sinkende Wirkungsgrad im Reaktionsmedium kann ebenfalls durch die kontinuierliche und/oder getagte Einbringung weiterer Wirksubstanzen ausgeglichen werden.

Zum Einbringen der fluiden Nährstoffe oder Oxidationsmittel wird an der Unterseite der Reaktorelemente über steuerbare Ventile eine Zufuhrmöglichkeit geschaffen. Durch die mäanderartige Führung des Reaktionsmediums und/oder durch die aufsteigenden fluiden Wirkstoffe, wie die Gasbläschen, wird für eine gute Durchmischung und Verteilung innerhalb des gesamten Reaktors gesorgt. Natürlich können auf diese Weise auch gasförmige Nährstoffe, Oxidationsmittel oder Wirkstoffe eingebracht werden.

Die eingebrachten Gase bewirken, durch das Aufsteigen der Gasbläschen, ein Selbstreinigen der Reaktorinnenfläche. Eine Entnahmestelle für Proben, um den Prozessfortschritt zu überprüfen, ist ebenfalls unten am Reaktorelement vorgesehen.

Gemäß einem weiteren besonderen Merkmal der Erfindung sind die Reaktorelemente an der Oberseite mit einem, vorzugsweise abnehmbaren, Abschlusselement verschlossen und zur Ausbringung von gasförmigen Prozessprodukten, wie beispielsweise Sauerstoff oder CO₂, vorzugsweise während des Prozesses, ist ein Abluftsystem vorgesehen, das über der Reaktionsmediums-Oberfläche bzw. über der Oberseite der Reaktorelemente vorgesehen ist und gegebenenfalls ist zur Ausbringung von gasförmigen Prozessprodukten eine über der Reaktionsmediums-Oberfläche bzw. über der Oberseite der Reaktorelemente vorgesehene Sammeleinrichtung mit dem Abluftsystem vorgesehen. Gasförmige Prozessprodukte, wie Stoffwechselprodukte, die im photosynthetischen oder photochemischen Prozess entstehen, können durch die Druckfreiheit im Reaktorelement frei im Reaktionsmedium aufsteigen.

Sauerstoff der im photosynthetischen Prozess entsteht und der phototrophische Mikroorganismen schädigt und Prozessabgase, die im photochemischen Prozess entstehen, können durch die Druckfreiheit im Reaktorelement frei im Reaktionsmedium aufsteigen. Durch die ganz oder teilweise nach oben hin offene Konstruktion des Reaktorelementes ist ein Entweichen und/oder Absaugen des Sauerstoffes möglich.

Die Prozess-Abgas-Ausbringung wird durch die im Prozess entstehenden, aufsteigenden Bläschen gefördert und/oder durch zusätzlich eingeblasene Gase gegebenenfalls gesteuert.

Zur Ausbringung von gasförmigen Prozessprodukten ist eine über der Reaktionsmediums-Oberfläche bzw. über der Oberseite der Reaktorelemente vorgesehene Sammeleinrichtung mit dem Abluftsystem vorgesehen. Dadurch können die gasförmigen Prozessprodukte gesammelt und gegebenenfalls einer weiteren Verwertung oder Entsorgung zugeführt werden. Auch ein Verlust von Reaktionsmedium durch Verdunstung und/oder durch Spritzverlust und ein kontrolliertes Austragen und Sammeln von Gasen ist durch eine geschlossen Bauweise möglich.

Vor dem Einlass, insbesondere vor der Einlassöffnung, und/oder nach dem Auslass, insbesondere nach der Auslassöffnung, kann ein Siphon vorgesehen sein. Das Reaktionsmedium kann durch einen Siphon dem ersten Reaktorelement druckfrei bzw. drucklos zugeführt werden und durch einen weiteren Siphon nach dem Reaktor, drucklos und gegebenenfalls gasdicht abgeführt werden.

Gemäß einem besonderen Merkmal der Erfindung ist, vorzugsweise an der Oberseite des Biosolarreaktors, mindestens ein Füllstandssensor angeordnet, wobei der Füllstandssensor mit einem Nachfüllsystem verbunden ist. Dadurch wird der Füllstand des Reaktionsmediums immer auf seinen vordefinierten Wert gehalten. Ein optimaler Prozess wird damit gewährleistet.

Nach einer besonderen Weiterbildung der Erfindung ist der Reaktor über mindestens eine Achse zur Lichteinstrahlung verstellbar. Da phototropische Mikroorganismen nur in der Zone nah an der Oberfläche einen optimalen photosynthetischen Prozess durchlaufen und zur Nahrungsaufnahme und zur Teilung durch zu viel UV-Strahlung beeinträchtigt werden, ist es von Vorteil, innerhalb des Reaktorelementes sowohl an die Außenzone als auch ins Innere geführt zu werden.

Zu intensives, direkt einstrahlendes UV-Licht schädigt oder beeinträchtigt das Wachstum der Mikroorganismen und erhöht die Temperatur des Reaktionsmediums über das ideale Maß, welches wieder gekühlt werden muss. Durch die Durchmischung des Reaktionsmediums gelangen alle phototrophischen Mikroorganismen ausreichend an die mit Licht durchflutete, außenwandnahe Lichtzone des Reaktorelementes. Bei der photokatalytischen Oxidation ist es eben von Vorteil, wenn alle Moleküle innerhalb des Reaktorelementes an die Licht durchflutete, außenwandnahe Lichtzone des Reaktorelementes geführt werden.

Eine Parallelstellung zur Lichtquelle bzw. ein paralleles Folgen der Sonneneinstrahlung des Reaktors wird meist ausreichen und somit wird eine massiv bessere Platznutzung möglich.

Weiters wird parallel eingestrahltes Licht von der Reaktoroberfläche teilweise reflektiert und steht dem gegenüberliegenden Reaktor zur Verfügung.
Bei insbesondere senkrechter Sonnenstrahlung, schlechter geografischer Lage oder bei besonders Licht bedürftigen phototropischen Mikroorganismen oder photokatalytischen Prozessen kann eine der Lichtquelle zugewandte Stellung des Reaktors in einem beliebigen Winkel gewählt werden.

Eine weitere Aufgabe der Erfindung liegt darin, einen Biosolarreaktor für eine prozessoptimierte Anlage im industriellen Betrieb zu schaffen.

Die Aufgabe der Erfindung wird durch den nachstehend aufgezeigten Biosolarreaktor gelöst.

Der erfindungsgemäße Biosolarreaktor ist dadurch gekennzeichnet, dass die einzelnen Reaktorelemente an der Unterseite an jeweils den äußersten Röhren bzw. Kammern angeordnete Ein- und Auslassöffnungen aufweisen, wobei der Einlassöffnung ein, gegen die Richtung der Schwerkraft, aufsteigender Ast, und der Auslassöffnung ein, in Richtung der Schwerkraft, abfallender Ast zugeordnet ist, dass alle Anschlüsse, insbesondere die Einlassöffnung, die Auslassöffnung und der Einbringungseinlass, mit dem Reaktionsmedium umspült werden, dass vorzugsweise die Einlass- und Auslassöffnung, vorzugsweise an der tiefsten Stelle des Reaktorelementes, insbesondere an den Röhren oder Kammern, angeordnet sind und dass gegebenenfalls an der Oberseite der Reaktorelemente ein Abluftsystem angeordnet ist.

Dabei ist eine Ausbildung von Vorteil, bei der die Einlass- und Auslassöffnung vorzugsweise an der Stirnseite des Reaktorelementes, insbesondere an den Röhren oder Kammern, angeordnet ist. Dadurch wird ein einfacher Aufbau erzielt und die Leitungen können entsprechend sicher verlegt werden.

Nach einer vorteilhaften Ausbildung der Erfindung sind die einzelnen Kammern bzw. Röhren zu einem mäanderförmigen Verlauf verbunden, wobei die zwischen den Kammern bzw. Röhren angeordneten Trennwände entsprechend verkürzt ausgebildet sind. Dadurch wird in vorteilhafter Weise erreicht, dass der Weg für den Flüssigkeitstransport vom Einlass zum Auslass wesentlich verlängert wird, so dass das Reaktionsmedium ausreichend lange im Reaktor verbleibt, um ein optimales Wachstum zu erzielen.

Gemäß einer besonderen Ausgestaltung sind mehrere Reaktorelemente über eine Halterung zu einem Reaktorpanel verbunden. Dadurch wird eine optimale Nutzung der Anlage gewährleistet.

Die Erfindung wird an Hand von Ausführungsbeispielen, die in der Zeichnung dargestellt sind, näher erläutert.

Es zeigen:
Fig. 1 eine schematische Darstellung eines Biosolarreaktor bestehend aus Röhren,
Fig. 2 eine Draufsicht gemäß Fig. 1,
Fig. 3 eine schematische Darstellung des weiteren Biosolarreaktor bestehend aus Stegplatten,
Fig. 4 eine Draufsicht gemäß Fig. 3,
Fig. 5 eine schematische Darstellung eines Seitenrisses einer Anlage mit einem Biosolarreaktor,
Fig. 6 eine schematische Darstellung in Draufsicht einer Anlage, bei der mehrere Biosolarreaktoren zusammen geschaltet sind.

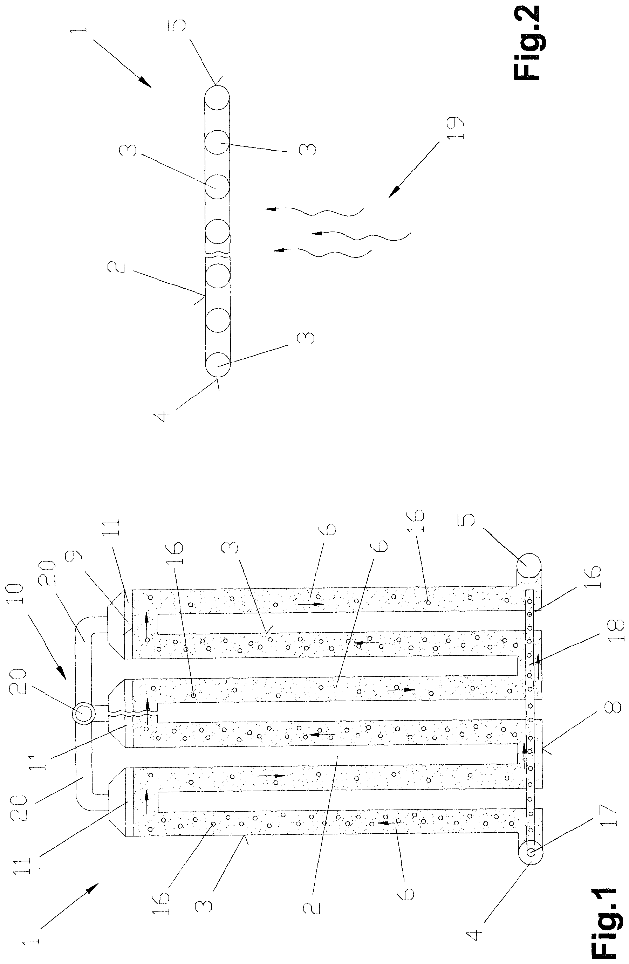

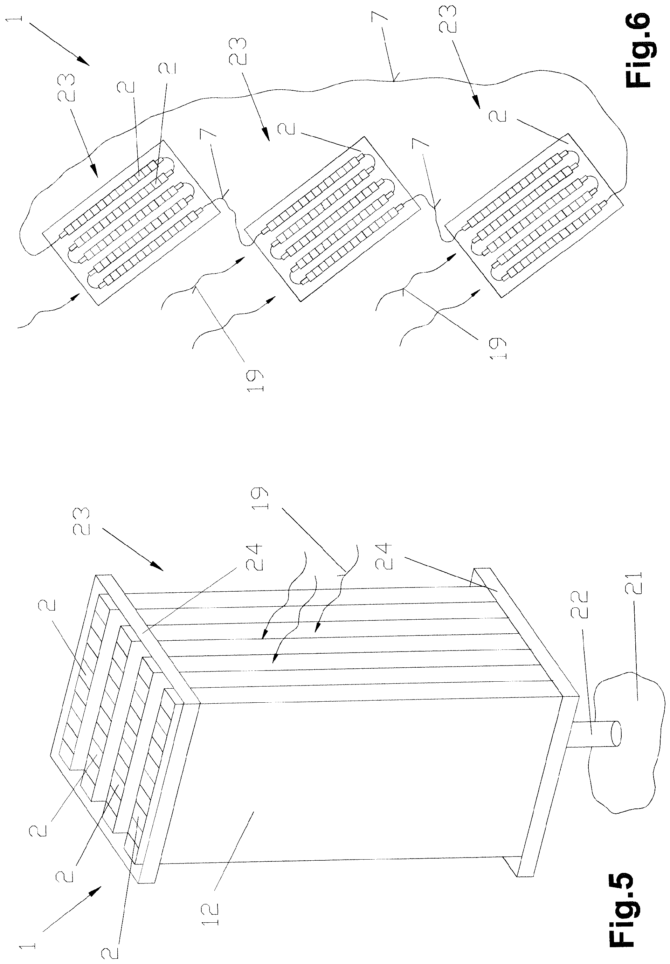

Gemäß der Fig. 1 und 2 besteht ein Reaktor, insbesondere ein Biosolarreaktor 1, aus mindestens einem Reaktorelement 2, das aus zumindest zwei aufrechten, unten und oben verbundenen Röhren 3 gebildet ist. Für den Aufbau eines Reaktorelementes 2 werden eine Vielzahl an Röhren 3 in Serie geschaltet. Vorzugsweise werden zur Bildung eines Reaktorelementes 2 siebzig Röhren 3 zusammen gefügt, wobei ein mäanderförmiger Verlauf durch die Röhren 3 gebildet wird, das heißt, dass sich immer ein aufsteigender Ast mit einem abfallenden Ast abwechseln und auf der Unterseite und Oberseite ein Übergang auf die weitere Röhre 3 vorhanden ist. Jeweils an der ersten und letzten Röhre 3 ist eine Einlassöffnung 4 und Auslassöffnung 5 auf der der Aufstellfläche zugordneten Seite der horizontal aufgestellten Röhren 3 angeordnet. Gleichzeitig werden zur Bildung des Biosolarreaktors 1 mehrere Reaktorelemente 2 seriell hintereinander geschaltet bzw. miteinander verbunden, wie nachstehend in Fig. 6 schematisch ersichtlich. Hierbei ist es möglich, dass alle Reaktorelemente 2 gleich ausgebildet sind oder aber auch, dass ein oder mehrere Reaktorelemente 2 unterschiedlich, insbesondere in der Anzahl der angeordneten Röhren 3, aufgebaut sind, das heißt, dass beispielsweise das erste Reaktorelement 2 mit 50 Röhren 3 aufgebaut ist und das weitere bzw. die weiteren dahinter parallel angeordneten Reaktorelemente 2 zum Beispiel 52, 54 usw. Röhren 3 aufweisen.

Ein derartiger Biosolarreaktor 1 wird für ein Verfahren für einen photochemischen, wie photokatalytischen und/oder photosynthetischen Prozess, insbesondere für eine Zucht und Produktion bzw. Hydrokultivierung von, vorzugsweise phototrophen, Mikroorganismen eingesetzt. Für seinen Betrieb wird der Biosolarreaktor 1 mit einem Reaktionsmedium 6, beispielsweise einer wässerigen Lösung oder einer Suspension aus Mikroorganismen und Nährlösung, gefüllt. Im Betrieb wird der Biosolarreaktor 1 nur mehr über seine ersten Einlassöffnung 4 gespeist. Die Führung bzw. Flussrichtung des Reaktionsmediums 6 erfolgt senkrecht einmal von unten nach oben, also entgegen der Schwerkraft und von oben nach unten, also in Richtung der Schwerkraft in dem Reaktorelement 2, also mäanderförmig durch die einzelnen Röhren3 eines Reaktorelementes 2. Bei einer Hintereinanderschaltung von mehreren Reaktorelementen 2, die miteinander verbunden sind, fließt das Reaktionsmedium 6 mäanderförmig durch den Reaktorelemente 2 und tritt dabei bei der Auslassöffnung 5 an der letzten Röhre 3 des Reaktorelementes 2 aus und wird über eine Verbindungsleitung 7, wie in Fig. 6 gezeigt, mit der Einlassöffnung 4 des nächsten Reaktorelementes 2 verbunden. Sowohl die Einbringung bzw. Einspeisung als auch die Ausbringung des Reaktionsmediums 6 in bzw. aus dem Biosolarreaktor 1, erfolgt vorzugsweise kontinuierlich, drucklos und frei zur Atmosphäre. Es ist dabei auch möglich, dass eine eigene Befüllöffnung (nicht dargestellt) vorhanden sein kann oder dass die Befüllung über eine der Einlassöffnungen 4 erfolgt. Die Einlassöffnung 4 und Auslassöffnung 5 sind zur besseren Handhabung immer auf der Unterseite 8 einer Röhre 3 angeordnet, wogegen eine Befüllöffnung parallel zur Einlassöffnung 4 oder auf der Oberseite 9 angeordnet ist. Wird die Anlage als geschlossenes System aufgebaut, so ist es bei der Befüllung erforderlich, dass eine Entlüftungsöffnung oder ein Abluftsystem 10 angeordnet ist, bei der die in den Reaktorelementen 2 enthalten Luft oder Gase entweichen kann bzw. abgesaugt wird. Vorzugsweise ist das Abluftsystem 10 auf der Oberseite 9 angeordnet, da die Luft im Reaktionsmedium 6 aufsteigt und somit einfach nach oben hin entweichen kann.

Die Reaktorelemente 2 sind somit mäanderartig als kommunizierende Röhren 3 miteinander verbunden, wobei die Einlassöffnung 4 und die Auslassöffnung 5 immer auf der Unterseite 8 der Reaktorelemente 2, insbesondere der Aufstellfläche zugewandten Seite, liegen bzw. angeordnet sind. Die Reaktorelemente 2 sind auf der Oberseite 9 mit einem Abschlusselement 11 verschlossen, wobei das Abschlusselement 11 abnehmbar ausgebildet ist, um beispielsweise die Anlage einfach Reinigen zu können.

Unter Ausnutzung des hydrostatischen Druck- und Niveauausgleichs erfolgt über die Einspeisung von Reaktionsmedium 6 an der Einlassöffnung 4 eine Strömung des Reaktionsmediums 6, das heißt, dass die einzelnen Reaktorelemente 2 an der Unterseite 8 an jeweils den äußersten Röhren 3 angeordnete Einlass- 4 und Auslassöffnungen 5 aufweisen, wobei der Einlassöffnung 4 jeweils eine gegen die Richtung der Schwerkraft, also ein aufsteigender Ast, und der Auslassöffnung 5 eine in Richtung der Schwerkraft, also ein abfallender Ast, zugeordnet ist und dass an der gegenüberliegenden Oberseite 9 der Reaktorelemente 2 das Abluftsystem 10 angeordnet ist, so dass aufsteigende Gase über das Abluftsystem 10 entweichen oder abgesaugt werden können. Für das Verfahren bedeutet das, dass für die Mikroorganismen eine stressfreie Strömung des Reaktionsmediums 6 ohne Pumpvorrichtungen erzeugt wird, so dass es zu keinen Beschädigungen der Reaktionsmedium 6, insbesondere der darin enthaltenen Algen, kommen kann und der Ertrag einer derartigen Anlage wesentlich erhöht wird. Dadurch wird ein freies Fließen zwischen den einzelnen Reaktorelementen 2 ermöglicht, ohne das weitere Energie zugeführt werden muss.

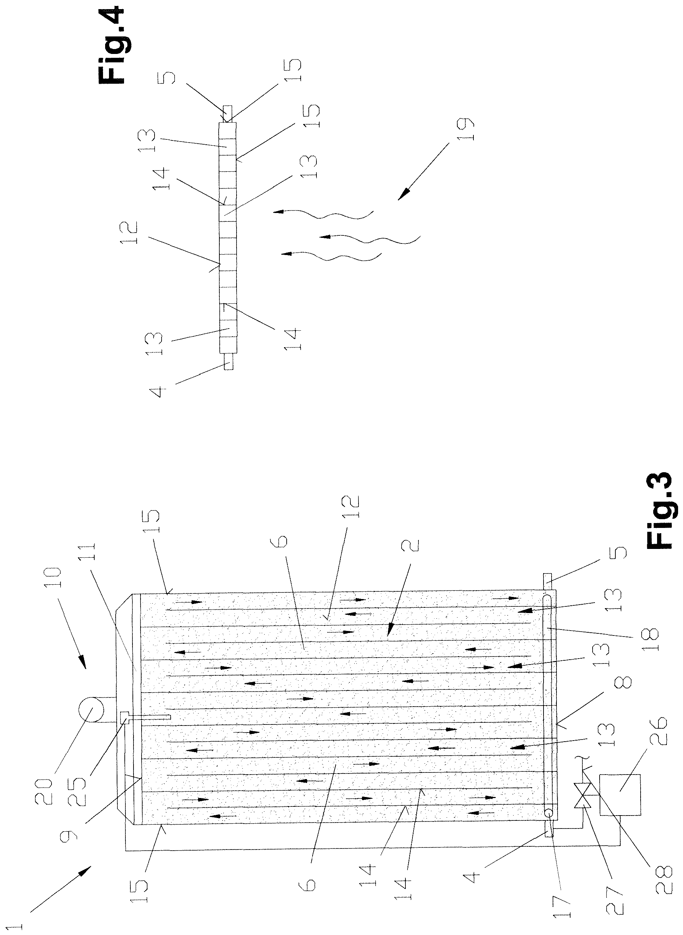

Gemäß den Fig. 3 und 4 ist eine alternative Bauart eines Biosolarreaktors 1 aufgezeigt. Dieser Biosolarreaktor 1, besteht aus Steg- bzw. Stegmehrfachplatten 12. Bei dieser Bauart besteht ein Reaktorelement 2 aus zumindest zwei, vorzugsweise rechteckigen, aufrechten, aus den Steg- bzw. Stegmehrfachplatten 12 gebildeten Kammern 13, wobei eine Seite der Kammer 8 als Trennwand 14 ausgebildet ist. Sowohl die Einlassöffnung 4 für die Einbringung bzw. Einspeisung als auch die Auslassöffnung 5 ist am unteren äußersten Reaktorrand, insbesondere an den Stirnflächen der Stegmehrfachplatten 12, vorgesehen, das heißt, dass die Kammern 13 den Röhren 3 entspricht und dass durch eine aneinander Reihen mehrere Kammern 13 bzw. Röhren 3 ein Reaktorelement 2 bzw. die Steg- bzw. Stegmehrfachplatte 12 gebildet wird, wobei für den Biosolarreaktor 1 vorzugsweise mehrere Reaktorelemente 2 hinter einander geschaltet sind, wie dies in Fig. 5 und 6 ersichtlich ist.

Um einen mäanderförmigen Verlauf der Kammern 13 zu erreichen, ist bei einer Verbindung von zwei oder mehr Kammern 13 deren Trennwand 14 bei einem aufsteigenden Ast niedriger als die Trennwand 14 zwischen den Röhren 3 bzw. Kammern 13 bei einem abfallenden Ast ausgebildet, das heißt, dass bei einem aufsteigenden Ast die Trennwand 14 auf der Oberseite 9 gegenüber den äußeren Wandteilen 15 der Kammer 13 gekürzt ist, wogegen bei einem abfallenden Ast die Trennwand 14 auf der Unterseite 8 kürzer ausgebildet ist, also die äußeren Wandteile 15 der Kammer 13. Dadurch entsteht ein Überlauf bzw. eine kommunizierende Öffnung, wenn der Flüssigkeitsstand in den Reaktorelementen 2 höher als die Trennwand 14 zwischen den Reaktorelementen 2 ist. Der Energieverbrauch wird minimiert, indem auf Pumpen zwischen den Prozessschritten weitestgehend verzichtet wird und beliebig viele gleich oder verschiedene Prozessschritte in selber Durchflusshöhe aneinander gekoppelt werden können.

Die einzelnen Reaktorelemente 2 sind vorzugsweise transparent oder transluzent oder bei Bedarf auch lichtdicht ausgeführt. Als Materialien können sowohl Glas oder UV-durchlässiger Kunststoff, wie z.B. Polymethylmethacrylat Verwendung finden. Die Verbindungen von Reaktorelement 2 zu einem weiteren Reaktorelement 2 befinden sich nun an der Unterseite 8 der Platte, insbesondere des Steg- Stegmehrfachplatte 12 des Reaktorelementes 2. Die Einlassöffnung 4 und die Auslassöffnung 5 sind so angelegt und ausgefräst, dass jeweils an der Unterseite 8 der äußersten Kammer 13 die Einlassöffnungen 4 und Auslassöffnungen 5 für Schlauchverbinder bzw. Verbindungleitungen 7 zur nächsten Reaktorelemente 2 angeordnet sind. Die Kammer 13 am Flüssigkeitseinlass, also mit der Einlassöffnung 4, ist dabei ein aufsteigender Ast bei der das Reaktionsmedium 6 entgegen der Schwerkraft zur Oberseite 9 fließt, wogegen die Kammer 13 am Auslass, also an der Auslassöffnung 5, ein abfallender Ast, bei dem das Reaktionsmedium 6 von der Oberseite 9 zur Unterseite 8 in Richtung der Schwerkraft strömt, zugeordnet ist.

Die Befüllung sowie der Betrieb des Biosolarreaktors 1 erfolgen analog den Ausführungen zu den Fig. 1 und 2, dabei wird beispielsweise zuerst das Reaktionsmedium 6 mit einem Befüllungsrohr stressfrei in den Biosolarreaktor 1 gefüllt, indem das Reaktionsmedium 6 von einem Aufbewahrungsbehälter (nicht dargestellt), der über den Niveau der Oberseite 9 des Biosolarreaktors 1 angeordnet ist, von dem Aufbewahrungsbehälter über das Befüllungsrohr an den unteren Einlassöffnung 4 angeordnet ist, so dass das Reaktionsmedium 6 ohne Pumpen und somit ohne Beschädigungen, in die Reaktorelemente 2 fließen kann. Grundsätzlich ist es jedoch auch möglich, dass bei dem Befüllvorgang eine Pumpe eingesetzt wird und anschließend die Reaktorströmung pumpenlos erfolgt.

In Hinblick auf die Lichteinstrahlung auf die Reaktorelemente 2 - auf die noch später näher eingegangen wird, ist ein geneigter Reaktor (nicht dargestellt) möglich. Trotzdem, dass der Reaktor in einem Winkel geneigt ist, fließt das Reaktionsmedium 6 einmal von oben nach unten bzw. in Richtung Schwerkraft und von unten nach oben bzw. gegen die Richtung der Schwerkraft. Wesentlich ist, dass die Reaktorelemente 2 senkrecht oder ein einem zur Senkrechten leicht geneigten Winkel aufgestellt sind, wobei die Reaktorelemente 2 eine Länge zwischen 15 und 30m, insbesondere 19m, aufweisen und die Breite der Reaktorelemente 2 aufgrund der Größe der Kammer 13 bzw. der Röhren 3 und deren Anzahl bestimmt ist. vorzugsweise werden 70 Kammern 13 nebeneinander angeordnet. Man kann also sagen, dass die Reaktorelemente 2 vorzugsweise 19 Meter in die Höhe ragen, so dass zur Stabilisierung entsprechende Vorkehrungen benötigt werden.

Gemäß den Ausführungsbeispielen der Fig. 1 und 2 und der Fig. 3 und 4 ist für die, kontinuierliche oder chargenweise, Einbringung von Zusatzstoffen 16 (in Fig. 1 dargestellt), wie beispielsweise Nährstofflösungen bzw. -gasen und/oder Oxidationsmittel und/oder Wirksubstanzen und/oder den Prozess fördernde gelöste Stoffe bzw. Gase, vorzugsweise während des Prozesses, an der Unterseite 8 des Reaktors, im Bereich der Umlenkung des Reaktionsmediums 6, mindestens ein Einbringungseinlass 17, beispielsweise ein steuerbares Ventil, vorgesehen, wie dies schematisch dargestellt ist Dabei ist es selbstverständlich möglich, dass der Einbringungseinlass 17 mit der Einlassöffnung 4 kombiniert ist. Der Einbringungseinlass 17 ist vorzugsweise mit einem Versorgungsrohr 18 verbunden, durch das die Zusatzstoffe 16 in die einzelnen Kammern 13 oder Röhren 3 verteilt wird.

Entsprechend dem Verfahren wird das Reaktionsmedium 6 wahlweise vor dem Eintritt in den Reaktor mit CO₂ oder anderen Gasen gesättigt. Der Sättigungsgrad wird den Bedürfnissen des Prozesses entsprechend angereichert und/oder während des Verweilens im Reaktor mit CO₂ oder anderen Gasen versorgt. Der im photosynthetischen Prozess, durch stetes Heranwachsen der Mikroorganismen, sinkende CO₂ Gehalt im Reaktionsmedium 6 kann durch die kontinuierliche oder getagte Einbringung von CO₂ ausgeglichen werden.

Durch die Einbringung der Zusatzstoffe 16 am unteren Ende der Flüssigkeitssäule über die Einbringungseinlässe 17 und das Versorgungsrohr 18 gemäß der Fig. 1 und 3 erfolgt eine Durchmischung und gleichmäßige Verteilung der Zusatzstoffe 16 im Reaktionsmedium 6, wie schematisch dargestellt.

Das Einbringen von Zusatzstoffen 16, wie Fluide und Gase optimiert weiters die Versorgung mit Licht, da durch die somit entstehende Verwirbelung im Reaktionsmedium 6 alle Moleküle oder phototropischen Mikroorganismen ausreichend an die mit Licht durchflutete, außenwandnahe Lichtzone - angedeutet mit den Pfeilen 19 - des Reaktorelementes 2 geführt werden.
Die Einbringung von Fluiden und Gasen erzeugt eine Verwirbelung im Reaktionsmedium 6, wodurch ein weiterer vorteilhafter Effekt zum Tragen kommt, nämlich dass durch das Aufsteigen der Gasbläschen eine kontinuierliche Reinigung der Reaktorinnenflächen bewirkt wird.

Ferner kann auch durch definiert eingebrachten Fluide und Gase eine Erwärmung oder Abkühlung des Reaktionsmediums 6 erfolgen. Die eingebrachten Zusatzstoffe 16 können somit zum kontrollierten Temperieren des Reaktionsmediums 6 herangezogen werden.

Es ist auch eine Ausbildung des Biosolarreaktor 1 mit einer Archimedischen Schraube (nicht dargestellt) möglich. Die Archimedische Schraube oder eine Spirale nach Da Vinci dient zum Transport des Reaktionsmediums 6 sowohl innerhalb des Reaktors als auch zwischen Reaktorteilen oder Reaktoren. Vor dem Einlass und nach dem Auslass ist je ein Siphon vorgesehen. Natürlich können die Siphone auch unabhängig von der Archimedischen Schraube vor dem Einlass bzw. nach dem Auslass aus dem Reaktor angeordnet werden. Das Reaktionsmedium 6 kann durch einen Siphon dem ersten Reaktorelement 2 druckfrei bzw. drucklos zugeführt werden, wobei dies ebenfalls bei den Ausführungen der Fig. 1 bis 6 möglich ist. Die Archimedische Schraube oder eine Spirale nach Da Vinci kann bei dem Verfahren für kontinuierliche photokatalytische und photosynthetischen Prozesse und Transporte in Biosolarreaktoren 1 eingesetzt werden. Insbesondere dann, wenn der Transport des Reaktionsmediums 6 die Überwindung von Höhendifferenzen verlangt. Mit einem Einsatz der Archimedischen Schraube oder der Spirale nach Da Vinci gelingt ein- oder auch mehrmaliger stressfreier Transport oder kann zum Befüllen der Anlage verwendet werden. Für folgende Anwendungen könnte diese Einrichtung zum Einsatz kommen:
- Transport, zum mehrmaligen Durchlaufen des Reaktionsmediums 6, durch denselben Reaktor.
- Transport zwischen einer Reihe von, gegebenenfalls verschiedenen, Reaktoren und/oder Reifungstanks, die einmal oder mehrmals, durchlaufen werden.
- Ein oder mehrmaliges Transportieren eines Reaktionsmediums 6 wechselweise zwischen einem Tank und einem beliebigen Bioreaktor.
- Ein oder mehrmaliger Transport eines Reaktionsmediums zwischen Tanks.

Um einen möglichst hohe Ertrag der Anlage zu erreichen, ist ein druckfreie bzw. drucklose Transport des Reaktionsmediums 6 von Vorteil, da dadurch die Algen bzw. das Reaktionsmedium 6 nicht zerstört bzw. beschädigt wird und somit das Wachstum nicht behindert wird, das heißt, dass das Reaktionsmedium 6 während des gesamten Transports keinem weiteren Druck, als jenem ausgesetzt ist, der innerhalb des Transportelementes durch das Eigengewicht des Reaktionsmediums 6 entsteht. Durch eine geringe Drehzahl wird das Reaktionsmedium 6 keinen nennenswerten Zentrifugalkräften ausgesetzt. Die Entwicklung der Mikroorganismen oder der Ablauf des Prozesses wird durch den Transport nicht unterbrochen oder gestört. Die Prozesse können frei von Stress, Beschleunigung und Druck ablaufen, so dass das Wachstum der Algen bzw. Mikroorganismen nicht unterbrochen wird. Das Reaktionsmedium 6 ist während des gesamten Transports keiner höheren Appression, als jener ausgesetzt, der innerhalb des Transportelementes durch das freie Fließen des Reaktionsmediums entsteht. Die Entwickelung der Mikroorganismen oder der Ablauf des Prozesses wird durch den Transport nicht unterbrochen oder gestört. Abriebverletzungen und Beschädigung der Zellwände der Mikroorganismen oder Moleküle wie durch Pumpen werden ausgeschlossen.

Durch die Nutzung des hydrostatischen Druckausgleiches in einer Archimedischen Schraube oder in einer Spirale nach Da Vinci bleibt die Appressionsfreiheit gewahrt.

Zur Ausbringung von gasförmigen Prozessprodukten, wie beispielsweise Sauerstoff, vorzugsweise während des Prozesses, ist das Abluftsystem 10 vorgesehen, das über der Reaktionsmediums-Oberfläche bzw. über der Oberseite 9 der Reaktorelemente 2 vorgesehen ist. Zur Ausbringung dieser gasförmigen Prozessprodukte kann eine über dem Flüssigkeitsspiegel des Reaktionsmediums 6 bzw. über der Oberseite 9 der Reaktorelemente 2 vorgesehene Sammeleinrichtung 20 mit einer Absauganlage (nicht dargestellt) vorgesehen sein.

Bei senkrechter Sonneneinstrahlung auf den Reaktor, schlechter geografischer Lage oder bei besonders Licht bedürftigen Mikroorganismen oder photokatalytischen Prozessen kann der Biosolarreaktor 1 über mindestens eine Achse 22 zur Lichteinstrahlung verstellbar ausgeführt werden. Bei einer bevorzugten Ausführung kann der Biosolarreaktor 1 auf zwei Achsen 22 der Sonne nachgeführt werden, wie dies schematisch in Fig. 6 mit drei Reaktorelementen 2, die aus Steg- bzw. Stegmehrfachplatte 12 gebildet sind, dargestellt ist. Die Achse 22 kann in der Aufstellfläche bzw. im Boden 21 verankert sein.

Gemäß der Fig. 5 und 6 ist ein aus Reaktorelementen 2 gebildetes Reaktorpanel 23 derart angeordnet, dass die - schematisch angedeuteten - Licht- bzw. Sonnenstrahlen 19 in Richtung der Panelachse, also stirnseitig, auftreffen, wobei das Reaktorpanel 23 entsprechend den Sonnenstrahlen nachgeführt werden kann. Daraus ergibt sich, dass die Sonnenstrahlen 19 zwischen den einzelnen Reaktorelementen 2 eintreten können und somit eine sehr hohe Versorgung der Mikroorganismen in dem Reaktionsmedium 6 mit Licht, insbesondere den Sonnenstrahlen 19, gewährleistet ist. Ein bestmögliches Wachstum wird dadurch erreicht. Daraus ist auch ersichtlich, dass für den Flüssigkeitstransport innerhalb der Anlage keine Hilfsmittel, wie Pumpen, Motoren o. dgl. eingesetzt werden, wobei es jedoch möglich ist, dass vorzugsweise sogenannte Schaufelradpumpen zum schonenden Transport der Algen bzw. Mikroorganismen im Reaktionsmedium 6 eingesetzt werden. Vorzugsweise werden die Anlagen derart aufgebaut, dass die Reaktorpanele 23 hintereinander geschaltet werden, wobei der letzte Reaktorpanel 23 wiederum mit dem ersten Reaktorpanel 23 verbunden wird, um eine endlose Schleife zu erzielen. Damit kann das Reaktionsmedium 6 solange in den Reaktorelementen 2 bzw. Reaktorpanelen 23 bleiben, bis ein ausreichendes Wachstum erzielt wurde.

Gemäß der Fig. 6 sind mehrere, vorzugsweise miteinander über Verbindungsleitungen 7 verbundene, Reaktorpanele 23 vorgesehen, die derart angeordnet sind, dass die Licht- bzw. Sonnenstrahlen 19 parallel zu den Panelachsen 22 verlaufen und somit wiederum zwischen die Reaktorelemente 2 eindringen können..

Bei einer speziellen Ausführungsvariante sind die Reaktorelemente 2 aufrecht hängend und/oder stehend in eine obere und/oder untere Halterung 24 (Fig. 5) eingebracht.
Diese Halterung 24 kann folgende Funktionen erfüllen.
- Die Funktion als Drehelement, um der Sonneneinstrahlung 19 zu folgen.
- Die Kippfunktion um das Reaktorelement 2 zur Sonne hin zu neigen.
- Den Reaktorelementen 2 halt zu geben.
- Die Reaktorelemente 2 mäanderartig miteinander zu verbinden.
- Die einzelnen Reaktorelemente gasdicht verschließen zu können.
Diese Halterung 24 kann, wenigstens zwei bis beliebig viele, Reaktorelemente 2 zu einem Reaktorpanel 23 aufnehmen.
Dies ermöglicht ein enges Stellen und oder hintereinander Stellen von Reaktoren, was eine maximale Platznutzung erlaubt.

Das Verfahren ermöglicht ein optimales kombinieren von Reaktionsphasen unter Licht und Ruhephasen im Dunkel sowie einen stressfreien Transport.
So wird ein Aufbau von kontinuierlich einmalig durchlaufenden Prozessen ermöglicht oder modular, gesteuertes, mehrfaches Durchlaufen der einzelnen Teile.

Das Reaktionsmedium 6 kann vor der eigentlichen Reaktion in einem Anreicherungstank mit Nährstoffen und Nährgasen grundversorgt werden, die die Bioreaktion von Beginn an begünstigen. Im Fall der Abwasserreinigung oder Schadstoffbeseitigung kann eine, den Mikroorganismen maximal zumutbare Erstanreicherung mit den jeweiligen Schadstoffen im Reaktionsmedium erzeugt werden.

Das Reaktionsmedium 6 kann ideal temperiert werden und die, dem Zweck der Reaktion entsprechenden, Mikroorganismen oder chemischen Stoffe können in einer definierten Menge eingebracht werden.

Zum Aufrechterhalten der idealen Reaktionsbedingungen können im Reaktionsmedium 6 Temperatur, Prozessfluidgehalt, Prozessgasgehalt, Umwälzung, Durchmischung, Lichtzufuhr, und Abfuhr von Stoffwechselprodukten kontrolliert und gesteuert werden. Da bei einem derartig geschlossen System durch die intensive Sonneneinstrahlung 19 es zum Verdampfen der Flüssigkeit, insbesondere des Wassergehaltes, im Reaktionsmedium 6 kommt, ist es erforderlich, den Füllstand ständig zu überwachen. Würde der Flüssigkeitsstand nämlich unterhalb der Trennwende 14 auf der Oberseite 9 kommen, so würde die Strömung des Reaktionsmediums 6 unterbrechen und stoppen. Damit dies verhindert wird, ist, vorzugsweise auf der Oberseite 9, ein Füllstandssensor 25, wie in Fig. 3 ersichtlich, angeordnet, der mit einem Flüssigkeitsversorgungssystem 26 verbunden ist. Sinkt das Reaktionsmedium 6 aufgrund der Verdunstung unterhalb eines voreinstellbaren Wertes ab, so erkennt dies der Füllstandssensor 25 und leitet ein entsprechendes Signal an das Flüssigkeitsversorgungssystem 26 weiter. Daraufhin wird beispielsweise ein Ventil 27 im Bereich der Einlassöffnung 4 angesteuert, so dass von einer Versorgungsleitung 28 zusätzliche Flüssigkeit, insbesondere Wasser, oder ein Reaktionsmedium 6, eingefüllt wird. Dazu ist es lediglich erforderlich, dass der Druck in der Versorgungsleitung 28 höher ist, als der Druck in den Reaktorelement 2 oder dass über eine Pumpe die zusätzliche Flüssigkeit oder das zusätzliche Reaktionsmedium 6 eingespeist wird.

Da derartige Reaktorelemente 2 beispielsweise eine Höhe zwischen 5 m und 15 m aufweisen und die Breite des Reaktorelementes zwischen 2 m und 2,5 m belaufen, ist ein spezieller Befüllvorgang notwendig, da durch den mäanderförmigen Verlauf der Flüssigkeit der Flüssigkeitsstand unterschiedlich hoch ausfallen kann, bevor der Füllstandssensor 25 den richtigen Füllstand anzeigt. Somit wird zum Befüllen immer nur eine bestimmte Menge zusätzlicher Flüssigkeit oder Reaktormedium 6 eingefüllt und anschließend eine definierte Zeitdauer abgewartet, bis der Füllstandssensor 25 abgefragt wird. Selbstverständlich ist es auch möglich, dass mehrere Flüssigkeitssensoren 25 pro Reaktorelemente 2 angeordnet sein können. Grundsätzlich ist es möglich, dass das Nachbefüllen über das Flüssigkeitsversorgungssystem 26 von einer zentralen Stelle oder über mehrere Ventile 27 einer Anlage erfolgen kann. Wesentlich ist, dass aufgrund des Plattenaufbaus und der mäanderförmigen Strömungsrichtung immer darauf geachtet wird, dass sich der Füllstand nicht sofort über den gesamten Bereich verändert, sondern eine bestimmte Zeit abgewartet wird, bevor eine konstante Anhebung des Flüssigkeitsstandes kommt und somit ein Überlaufen verhindert werden kann. Der Flüssigkeitssensor 25 kann dabei aus einem einfachen Schwimmer oder einen elektronischen Sensor bestehen, der in das Reaktionsmedium 6 eintaucht. Auch könnte bei einer Veränderung der Füllhöhe sich beispielsweise der Widerstand des Flüssigkeitssensors 25 verändern. Es ist auch möglich, dass auf der Unterseite 8 eine Art Drucksensor als Flüssigkeitssensor 25 eingesetzt wird, so dass der Druck der Flüssigkeitssäule überwacht wird.

Das oben aufgezeigte Verfahren löst nachstehende Problematiken in vorteilhafterweise:
- Kontinuierliche photokatalytische und photosynthetische Prozesse und Transporte in Solarreaktoren
- Kontrollierter und optimierter Energieverbrauch im Prozess
- Kontrollierte und optimierte Einbringung von Nährlösungen und prozessfördernden Lösungen
- Kontrollierte und optimierte Einbringung von Nähr- und Prozessgasen
- Kontrollierte und optimierte Reduktion von Schadstoffen
- Optimierte Ausbringung und Sammeln von gasförmigen Prozessprodukten
- Kontrollierte und optimierte Versorgung mit Licht
- Minimierung des Platzverbrauches durch Lichtführung
- Kontrollierte und optimierte Prozesstemperatur
- Stressfreier Transport des Reaktionsmediums 6.

Dabei ist es auch möglich, dass der Biosolarreaktor 1, insbesondere die Reaktorelemente 2, in einer lichtdurchlässigen Flüssigkeit (nicht dargestellt) angeordnet ist, d.h., dass die Reaktorelemente 2 von einer lichtdurchlässigen Flüssigkeit, wie beispielsweise Wasser, umspült werden.

Weiters ist es möglich, dass bei einer derartigen Anlage auch unterschiedlich aufgebaute Platten, insbesondere Reaktorelemente 2, eingesetzt werden können. Dazu können beispielsweise Platten gemäß der AT 506 373 B1, bei denen sämtliche Anschlüsse auf der Oberseite 9 angeordnet sind, mit den erfindungsgemäßen Platten, bei denen sämtliche Anschlüsse an der Unterseite 8 angeordnet sind, kombiniert werden. Hierzu könnte jedoch eine Pumpe zum Fördern des Reaktionsmediums 6 eingesetzt werden. Als Pumpe wird wiederum eine Schaufelradpumpe zum schonenden Transport der Mikroorganismen bzw. Algen im Reaktionsmedium 6 eingesetzt werden.

Grundsätzlich ist zu erwähnen, dass zum Erzeugen einer Strömung des Reaktionsmediums 6 eine - nicht dargestellte - Pumpe eingesetzt werden kann. Bei einer derartigen erfindungsgemäßen Einrichtung wird ein Biosolarreaktor 1, insbesondere ein Reaktorpanel 23, vorzugsweise aus 12 Platten, wobei eine Platte beispielsweise eine Höhe von ca. 6 m und eine Breite von ca. 2,10 m aufweist, gebildet, wobei das Reaktionsmedium 6 ca. 44 Stunden benötigt, um durch die gesamten Platten, bzw. Reaktorelemente 2 hindurch zu strömen.

## Patentansprüche

1. Verfahren für einen photochemischen, wie photokatalytischen und/oder photosynthetischen Prozess, insbesondere für eine Zucht und Produktion bzw. Hydrokultivierung, von, vorzugsweise phototrophen, Mikroorganismen, wobei ein Reaktionsmedium (6), insbesondere eine wässerige Lösung oder eine Suspension aus Mikroorganismen und Nährlösung, mäanderförmig in einem Reaktorelement (2), welches mindestens aus zwei aufrechten, verbundenen Röhren (3) oder Kammern (13) gebildet wird, geführt wird, wobei die mäanderförmige Führung des Reaktionsmedium (6) senkrecht oder in einem Winkel geneigt, erfolgt und vorzugsweise mehrere Reaktorelemente (2) seriell zu einem Biosolarreaktor (1) verbunden werden und unter Ausnutzung des hydrostatischen Druck- und Niveauausgleich im Biosolarreaktor (1) eine für die Mikroorganismen stressfreie Strömung des Reaktionsmediums (6) erzeugt wird, **dadurch gekennzeichnet, dass** an die einzelnen Reaktorelemente (2) an der Unterseite (8) an jeweils den äußersten Röhren (3) oder Kammern (13) Ein- und Auslassöffnungen (4, 5) angeordnet werden, wobei der Einlassöffnung (4) jeweils eine gegen die Richtung der Schwerkraft, also ein aufsteigender Ast und der Auslassöffnung (5) eine in Richtung der Schwerkraft, also ein abfallender Ast, zugeordnet wird und dass die Einlassöffnung (4), die Auslassöffnung (5) und ein Einbringungseinlass (17) für Zusatzstoffe im unteren Bereich mit dem Reaktionsmedium (6) umspült werden.

2. Verfahren nach Anspruch 1, **dadurch gekennzeichnet, dass** eine, kontinuierliche oder chargenweise, Einbringung von flüssigen und/oder gasförmigen Zusatzstoffen (16), wie insbesondere Nährstofflösungen und/oder Oxidationsmittel und/oder Wirksubstanzen und/oder den Prozess fördernde gelöste Stoffe, vorzugsweise während des Prozesses, an der Unterseite, im Bereich der Einlass- und/oder Auslassöffnungen (4, 5) des Reaktionsmediums (6), durchgeführt wird, wobei durch die Einbringung der Zusatzstoffe (16) am unteren Ende der Flüssigkeitssäule eine Durchmischung und gleichmäßige Verteilung der Zusatzstoffe (16) im Reaktionsmedium (6) erfolgt.

3. Verfahren nach Anspruch 1 oder 2, **dadurch gekennzeichnet, dass** über eine, vorzugsweise an der Oberseite des Biosolarreaktors (1) vorgesehene, Füllstandskontrolle der Füllstand des Reaktionsmediums (6) erfasst wird und bei Absinken durch Zugabe einer Flüssigkeit und/oder des Reaktionsmediums (6) auf einen voreinstellbaren Wert gehalten wird.

4. Verfahren nach einem oder mehreren der Ansprüche 1 bis 3, **dadurch gekennzeichnet, dass** die Ausbringung von gasförmigen Prozessprodukten, wie beispielsweise Sauerstoff oder CO₂, vorzugsweise während des Prozesses, über die Reaktionsmediums-Oberfläche erfolgt und vorzugsweise über ein Abluftsystem (10) an der Oberseite (9) des Reaktorelementes (2) abtransportiert bzw. abgesaugt wird.

5. Verfahren nach einem oder mehreren der Ansprüche 1 bis 4, **dadurch gekennzeichnet, dass** das Reaktorelement (2) auf der Oberseite (9) durch ein Abschlusselement (11), vorzugsweise abnehmbar, verschlossen wird.

6. Einrichtung zur Durchführung des Verfahrens nach einem oder mehreren der Ansprüche 1 bis 5, wobei ein Reaktor, insbesondere ein Biosolarreaktor (1), aus mindestens einem Reaktorelement (2) besteht, das aus mindestens zwei aufrechten, verbundenen Röhren (3) oder Kammern (13) gebildet ist, **dadurch gekennzeichnet, dass** die einzelnen Reaktorelemente (2) an der Unterseite (8) an jeweils den äußersten Röhren (3) oder Kammern (13) Ein- und Auslassöffnungen (4, 5) aufweisen, wobei der Einlassöffnung (4) jeweils eine gegen die Richtung der Schwerkraft, also ein aufsteigender Ast, und der Auslassöffnung (5) eine in Richtung der Schwerkraft, also ein abfallender Ast, zugeordnet ist, dass die Einlassöffnung (4), die Auslassöffnung (5) und ein Einbringungseinlass (17) für Zusatzstoffe im unteren Bereich mit dem Reaktionsmedium (6) umspült sind und dass vorzugsweise die Einlass- und/oder Auslassöffnung (4,5) an der tiefsten Stelle des Reaktorelementes (2) angeordnet ist.

7. Einrichtung nach Anspruch 6, **dadurch gekennzeichnet, dass** das Reaktorelement (2) aus mehreren, insbesondere siebzig vorzugsweise rechteckigen, aufrechten, Kammern (8) bzw. Röhren (3) gebildet ist, die durch eine Trennwand (14) voneinander getrennt sind, wobei die Trennwand (14) einmal auf der Oberseite (9) und anschließend an der Unterseite (8), also im Bereich eines Boden (21), für den Umlauf des Reaktionsmedium (6) offen ist.

8. Einrichtung nach Anspruch 6 oder 7, **dadurch gekennzeichnet, dass** bei einer Verbindung von zwei oder mehr Reaktorelementen (2) immer eine Auslassöffnung (4) mit einer Einlassöffnung (5), vorzugsweise über eine Verbindungsleitung (7), verbunden ist.

9. Einrichtung nach Anspruch 6 oder 8, **dadurch gekennzeichnet, dass** für die, kontinuierliche oder chargenweise, Einbringung von Zusatzstoffen (16), wie beispielsweise Nährstofflösungen bzw. -gasen und/oder Oxidationsmittel und/oder Wirksubstanzen und/oder den Prozess fördernde gelöste Stoffe bzw. Gase, vorzugsweise während des Prozesses, an der Reaktorunterseite, im Bereich der Umlenkung des Reaktionsmediums und/oder über die Verbindungsleitung (7), mindestens ein Einbringungseinlass (17) vorgesehen ist.

10. Einrichtung nach einem oder mehreren der Ansprüche 6 bis 9, **dadurch gekennzeichnet, dass** die Reaktorelemente (2) an der Oberseite (9) mit einem Abschlusselement (11), vorzugsweise abnehmbar, verschlossen sind und dass zur Ausbringung von gasförmigen Prozessprodukten, wie beispielsweise Sauerstoff oder CO2, vorzugsweise während des Prozesses, ein Abluftsystem (10) vorgesehen ist, das über der Reaktionsmediums-Oberfläche bzw. über der Oberseite (9) der Reaktorelemente (2) vorgesehen ist und dass gegebenenfalls zur Ausbringung von gasförmigen Prozessprodukten eine über der Reaktionsmediums-Oberfläche bzw. über der Oberseite (9) der Reaktorelemente (2) vorgesehene Sammeleinrichtung (20) mit dem Abluftsystem (10) vorgesehen ist.

11. Einrichtung nach einem oder mehreren der Ansprüche 6 bis 10, **dadurch gekennzeichnet, dass**, vorzugsweise an der Oberseite des Biosolarreaktors (1), mindestens ein Füllstandssensor angeordnet ist, wobei der Füllstandssensor mit einem Nachfüllsystem verbunden ist.

12. Einrichtung nach einem oder mehreren der Ansprüche 6 bis 11, **dadurch gekennzeichnet, dass** der Reaktor über mindestens eine Achse (22) zur Lichteinstrahlung (19) verstellbar ist.

13. Biosolarreaktor für einen photochemischen, wie photokatalytischen und/oder photosynthetischen Prozess, insbesondere für eine Zucht und Produktion bzw. Hydrokultivierung von, vorzugsweise phototrophen, Mikroorganismen, umfassend zumindest mehrere in Serie verbundene Reaktorelemente (2), wobei jedes Reaktorelement (2) aus mehreren, insbesondere siebzig, vorzugsweise rechteckigen, aufrechten, Kammern (13) bzw. Röhren (3) gebildet ist, in einem Halterahmen befestigt sind, **dadurch gekennzeichnet, dass** die einzelnen Reaktorelemente (2) an der Unterseite an jeweils den äußersten Röhren (3) bzw. Kammern (13) angeordnete Ein- und Auslassöffnungen (4, 5) aufweisen, wobei der Einlassöffnung (4) jeweils eine gegen die Richtung der Schwerkraft, also ein aufsteigender Ast, und der Auslassöffnung (5) eine in Richtung der Schwerkraft, also ein abfallender Ast, zugeordnet ist und dass die Einlassöffnung (4), die Auslassöffnung (5) und ein Einbringungseinlass (17) für Zusatzstoffe im unteren Bereich mit dem Reaktionsmedium (6)
umspült sind, wobei vorzugsweise die Einlass- und Auslassöffnung (4, 5), vorzugsweise an der tiefsten Stelle des Reaktorelementes (2), insbesondere an den Röhren (3) oder Kammer (13), angeordnet ist und dass an der Oberseite (9) der Reaktorelemente (2) ein Abluftsystem (10) angeordnet ist.

14. Biosolarreaktor nach Anspruch 13, **dadurch gekennzeichnet, dass** die einzelnen Kammern (13) bzw. Röhren (3) mäanderförmig ausgebildet sind, wobei die zwischen den Kammern (13) bzw. Röhren (3) angeordneten Trennwände (14) entsprechend verkürzt ausgebildet sind.

15. Biosolarreaktor nach Anspruch 13 oder 14, **dadurch gekennzeichnet, dass** mehrere Reaktorelemente (2) über eine Halterung (24) zu einem Reaktorpanel (23) verbunden sind.

## Claims

1. Method for a photochemical, e.g. photocatalytic and/or photosynthetic, process, in particular for breeding and production, or hydro cultivation, of preferably phototrophic microorganisms, a reaction medium (6), in particular an aqueous solution or a suspension of microorganisms and nutrient solution, being guided in a meandering manner in a reactor element (2) which is formed by at least two upright, connected tubes (3) or chambers (13), the reaction medium (6) being guided, in a meandering manner, perpendicularly or so as to be inclined at an angle, and preferably a plurality of reactor elements (2) being connected in series to form a biosolar reactor (1), and a reaction medium (6) flow that is stress-free for the microorganisms being generated by utilising the hydrostatic pressure and level compensation in the biosolar reactor (1), **characterised in that** inlet and outlet openings (4, 5) are arranged in the individual reactor elements (2), in the bottom face (8), in each of the outermost tubes (3) or chambers (13), a branch extending counter to the direction of gravity, i.e. an ascending branch, being associated with each inlet opening (4) and a branch extending in the direction of gravity, i.e. a descending branch, being associated with each outlet opening (5), and **in that** the reaction medium (6) flows through the inlet opening (4), the outlet opening (5) and an introduction inlet (17) for additives, in the lower region.

2. Method according to claim 1, **characterised in that** a continuous or batchwise introduction of liquid and/or gaseous additives (16), such as in particular nutrient solutions and/or oxidants and/or active substances and/or dissolved substances promoting the process, is carried out, preferably during the process, at the bottom, in the region of the inlet and/or outlet openings (4, 5) for the reaction medium (6), the introduction of the additives (16) at the lower end of the liquid column bringing about thorough mixing and even distribution of the additives (16) in the reaction medium (6).

3. Method according to either claim 1 or claim 2, **characterised in that** the fill level of the reaction medium (6) is detected by means of fill level monitoring that is preferably provided at the top of the biosolar reactor (1) and, when said level decreases, it is maintained at a presettable value by adding a liquid and/or the reaction medium (6).

4. Method according to one or more of claims 1 to 3, **characterised in that** gaseous process products such as oxygen or CO₂ are output over the reaction medium surface, preferably during the process, and said output products are preferably removed, or sucked out, by means of an air extraction system (10) at the top face (9) of the reactor element (2).

5. Method according to one or more of claims 1 to 4, **characterised in that** the reactor element (2) is closed on the top face (9) by means of a closure element (11), preferably in a detachable manner.

6. Device for carrying out the method according to one or more of claims 1 to 5, a reactor, in particular a biosolar reactor (1), consisting of at least one reactor element (2) that is formed by at least two upright, connected tubes (3) or chambers (13), **characterised in that** the individual reactor elements (2) comprise inlet and outlet openings (4, 5) in the lower face (8) in each of the outermost tubes (3) or chambers (13), a branch extending counter to the direction of gravity, i.e. an ascending branch, being associated with each inlet opening (4) and a branch extending in the direction of gravity, i.e. a descending branch, being associated with each outlet opening (5), **in that** reaction medium (6) flows through the inlet opening (4), the outlet opening (5) and an introduction inlet (17) for additives, in the lower region, and **in that** preferably the inlet and/or outlet opening (4, 5) is arranged at the lowest point of the reactor element (2).

7. Device according to claim 6, **characterised in that** the reactor element (2) is formed by a plurality of, in particular seventy, preferably rectangular, upright chambers (8) or tubes (3) which are separated from one another by a separating wall (14), the separating wall (14) being open once at the top (9) and subsequently at the bottom (8), i.e. in the region of a base (21), for circulation of the reaction medium (6).

8. Device according to either claim 6 or claim 7, **characterised in that**, when two or more reactor elements (2) are connected, an outlet opening (4) is always connected to an inlet opening (5), preferably via a connection line (7).

9. Device according to either claim 6 or claim 8, **characterised in that**, for the continuous or batchwise introduction of additives (16), such as nutrient solutions or gases and/or oxidants and/or active substances and/or dissolved substances or gases promoting the process, preferably during the process, at least one introduction inlet (17) is provided at the bottom of the reactor, in the region in which the reaction medium changes direction and/or via the connection line (7).

10. Device according to one or more of claims 6 to 9, **characterised in that** the reactor elements (2) are closed at the top face (9) by means of a closure element (11), preferably in a detachable manner, and **in that**, in order to output gaseous process products, such as oxygen or CO₂, preferably during the process, an air extraction system (10) is provided over the reaction medium surface or over the top face (9) of the reactor elements (2) and **in that**, in order to output gaseous process products, a collection device (20) over the reaction medium surface or over the top face (9) of the reactor elements (2) is optionally provided together with the air extraction system (10).

11. Device according to one or more of claims 6 to 10, **characterised in that** at least one fill level sensor is arranged, preferably at the top of the biosolar reactor (1), the fill level sensor being connected to a refill system.

12. Device according to one or more of claims 6 to 11, **characterised in that** the reactor can be adjusted by at least one shaft (22) for incident light irradiation (19).

13. Biosolar reactor for a photochemical, e.g. photocatalytic and/or photosynthetic, process, in particular for breeding and production, or hydro cultivation, of preferably phototropic microorganisms, including at least a plurality of reactor elements (2) connected in series, each reactor element (2) being formed by a plurality of, in particular seventy, preferably rectangular, upright chambers (13) or tubes (3), and being secured in a supporting frame, **characterised in that** the individual reactor elements (2) comprise inlet and outlet openings (4, 5) arranged in the bottom face in each of the outermost tubes (3) or chambers (13), a branch extending counter to the direction of gravity, i.e. an ascending branch, being associated with each inlet opening (4) and a branch extending in the direction of gravity, i.e. a descending branch, being associated with each outlet opening (5), and **in that** the reaction medium (6) flows through the inlet opening (4), the outlet opening (5) and an introduction inlet (17) for additives, in the lower region, the inlet and outlet opening (4, 5) preferably being arranged preferably at the lowest point of the reactor element (2), in particular in the tubes (3) or chambers (13), and **in that** an air extraction system (10) is arranged at the top face (9) of the reactor elements (2).

14. Biosolar reactor according to claim 13, **characterised in that** the individual chambers (13) or tubes (3) are formed in a meandering manner, the separating walls (14) arranged between the chambers (13) or pipes (3) being accordingly shortened.

15. Biosolar reactor according to either claim 13 or claim 14, **characterised in that** a plurality of reactor elements (2) are connected by means of a support (24) to form a reactor panel (23).

## Revendications

1. Procédé pour un processus photochimique, tel qu'un processus photo-catalytique et/ou photosynthétique, en particulier pour un élevage et une production ou une hydro-culture de micro-organismes, de préférence phototrophes, dans lequel un milieu de réaction (6), en particulier une solution aqueuse ou une suspension de micro-organismes et d'une solution nutritive, est guidé de manière sinueuse dans un élément de réacteur (2), lequel est formé d'au moins deux tubes (3) ou chambres (13) verticaux(verticales) relié(e)s, dans lequel le guidage sinueux du milieu de réaction (6) est réalisé verticalement ou en étant incliné selon un angle, et de préférence plusieurs éléments de réacteur (2) sont reliés en série de manière à obtenir un réacteur biosolaire (1) et, par l'utilisation de la compensation hydrostatique de pression et de niveau, un écoulement du milieu de réaction (6) sans stress pour les microorganismes est produit dans le réacteur biosolaire (1), **caractérisé en ce que** des ouvertures d'entrée et de sortie (4, 5) sont ménagées au niveau des différents éléments de réacteur (2), au niveau de la face inférieure (8), au niveau des tubes (3) ou chambres (13) les plus externes respectivement, dans lequel respectivement une branche allant dans le sens contraire de la gravité, par conséquent montante, est associée à l'ouverture d'entrée (4), et une branche allant dans le sens de la gravité, par conséquent une branche descendante, étant associée à l'ouverture de sortie (5), et **en ce que** l'ouverture d'entrée (4), l'ouverture de sortie (5) et l'entrée d'introduction (17) pour des additifs sont entourées par le milieu de réaction (6) dans la zone inférieure.

2. Procédé selon la revendication 1, **caractérisé en ce qu'**une introduction continue ou discontinue d'additifs (16) liquides et/ou gazeux, tels qu'en particulier des solutions d'éléments nutritifs et/ou des oxydants et/ou des substances actives et/ou des matières dissoutes favorisant le processus, est mise en oeuvre de préférence pendant le processus, au niveau de la face inférieure, dans la zone des ouvertures d'entrée et/ou de sortie (4, 5) du milieu de réaction (6), dans lequel l'introduction des additifs (16) à l'extrémité inférieure de la colonne de liquide permet d'obtenir un mélange et une répartition homogène des additifs (16) dans le milieu de réaction (6).

3. Procédé selon la revendication 1 ou 2, **caractérisé en ce que** le niveau du milieu de réaction (6) est détecté par l'intermédiaire d'un contrôle de niveau prévu de préférence au niveau de la face supérieure du réacteur biosolaire (1), et, en cas de baisse, est maintenu à une valeur pré-réglable par ajout d'un liquide et/ou du milieu de réaction (6).

4. Procédé selon l'une ou plusieurs des revendications 1 à 3, **caractérisé en ce que** l'émission de produits gazeux du processus, tels que par exemple l'oxygène ou le CO₂, a lieu, de préférence pendant le processus, au-dessus de la surface du milieu de réaction et est évacuée ou aspirée de préférence par l'intermédiaire d'un système d'évacuation d'air (10) au niveau de la face supérieure (9) de l'élément de réacteur (2).

5. Procédé selon l'une ou plusieurs des revendications 1 à 4, **caractérisé en ce que** l'élément de réacteur (2) sur la face supérieure (9) est fermé par un élément de fermeture (11), de préférence amovible.

6. Dispositif pour la mise en oeuvre du procédé selon l'une ou plusieurs des revendications 1 à 5, dans lequel un réacteur, en particulier un réacteur biosolaire (1), est constitué d'au moins un élément de réacteur (2), qui est formé d'au moins deux tubes (3) ou chambres (13) verticaux(verticales) relié(e)s, **caractérisé en ce que** les différents éléments de réacteur (2) présentent au niveau de la face inférieure (8), au niveau des tubes (3) ou chambres (13) les plus externes respectivement, des ouvertures d'entrée et de sortie (4, 5), dans lequel respectivement une branche allant dans le sens contraire de la gravité, par conséquent montante, est associée à l'ouverture d'entrée (4), et une branche allant dans le sens de la gravité, par conséquent une branche descendante, est associée à l'ouverture de sortie (5), **en ce que** l'ouverture d'entrée (4), l'ouverture de sortie (5) et une entrée d'introduction (17) pour des additifs sont entourées par le milieu de réaction (6) dans la zone inférieure et **en ce que** de préférence l'ouverture d'entrée et/ou de sortie (4, 5) est ménagée au point le plus profond de l'élément de réacteur (2) .

7. Dispositif selon la revendication 6, **caractérisé en ce que** l'élément de réacteur (2) est formé de plusieurs, en particulier de soixante-dix, chambres (8) ou tubes (3) verticales(verticaux) de préférence rectangulaires, qui sont séparé(e)s les un(e)s des autres par une cloison (14), dans lequel la cloison (14) est ouverte une fois sur la face supérieure (9) puis au niveau de la face inférieure (8), par conséquent dans la zone d'un fond (21), pour la circulation du milieu de réaction (6).

8. Dispositif selon la revendication 6 ou 7, **caractérisé en ce que**, dans le cas où deux ou plus de deux éléments de réacteur (2) sont reliés, une ouverture de sortie (4) est toujours reliée à une ouverture d'entrée (5), de préférence par l'intermédiaire d'une conduite de liaison (7).

9. Dispositif selon la revendication 6 ou 8, **caractérisé en ce que**, pour l'introduction continue ou discontinue d'additifs (16), tels que par exemple des solutions ou des gaz d'éléments nutritifs et/ou des oxydants et/ou des substances actives et/ou des matières dissoutes favorisant le processus ou des gaz, de préférence pendant le processus, au moins une entrée d'introduction (17) est prévue au niveau de la face inférieure du réacteur, dans la zone de la déviation du milieu de réaction et/ou par l'intermédiaire de la conduite de liaison (7).

10. Dispositif selon l'une ou plusieurs des revendications 6 à 9, **caractérisé en ce que** les éléments de réacteur (2) sont fermés au niveau de la face supérieure (9) par un élément de fermeture (11), de préférence amovible, et **en ce que**, pour l'émission de produits de processus gazeux, tels que par exemple l'oxygène ou le CO2, de préférence pendant le processus, un système d'évacuation d'air (10) est prévu, qui est prévu au-dessus de la surface du milieu de réaction ou au-dessus de la face supérieure (9) des éléments de réacteur (2) et **en ce que**, le cas échéant, pour l'émission de produits de processus gazeux, un dispositif collecteur (20) prévu au-dessus de la surface du milieu de réaction ou au-dessus de la face supérieure (9) des éléments de réacteur (2) et comprenant le système d'évacuation d'air (10) est prévu.

11. Dispositif selon l'une ou plusieurs des revendications 6 à 10, **caractérisé en ce qu'**au moins un capteur de niveau est agencé de préférence au niveau de la face supérieure du réacteur biosolaire (1), le capteur de niveau étant relié à un système de remplissage.

12. Dispositif selon l'une ou plusieurs des revendications 6 à 11, **caractérisé en ce que** le réacteur peut être déplacé sur au moins un axe (22) pour l'irradiation lumineuse (19).

13. Réacteur biosolaire pour un processus photochimique, tel qu'un processus photo-catalytique et/ou photosynthétique, en particulier pour un élevage et une production ou une hydro-culture de micro-organismes, de préférence phototrophes, comprenant au moins plusieurs éléments de réacteur (2) reliés en série, dans lequel chaque élément de réacteur (2) est formé de plusieurs, en particulier de soixante-dix, chambres (13) ou tubes (3) verticales (verticaux), de préférence rectangulaires, fixé(e)s dans un cadre de retenue, **caractérisé en ce que** les différents éléments de réacteur (2) présentent des ouvertures d'entrée et de sortie (4, 5) ménagées au niveau de la face inférieure (8), au niveau des tubes (3) ou chambres (13) les plus externes respectivement, dans lequel respectivement une branche allant dans le sens contraire de la gravité, par conséquent montante, est associée à l'ouverture d'entrée (4), et une branche allant dans le sens de la gravité, par conséquent une branche descendante, est associée à l'ouverture de sortie (5), et **en ce que** l'ouverture d'entrée (4), l'ouverture de sortie (5) et l'entrée d'introduction (17) pour des additifs sont entourées par le milieu de réaction (6) dans la zone inférieure, dans lequel l'ouverture d'entrée et/ou de sortie (4, 5) est ménagée de préférence au point le plus profond de l'élément de réacteur (2), en particulier au niveau des tubes (3) ou des chambres (13), et **en ce qu'**un système d'évacuation d'air (10) est agencé au niveau de la face supérieure (9) des éléments de réacteur (2).

14. Réacteur biosolaire selon la revendication 13, **caractérisé en ce que** les différentes chambres (13) ou les différents tubes (3) sont formé(e)s de manière sinueuse, dans lequel les cloisons (14) agencées entre les chambres (13) ou les tubes (3) sont raccourcies de manière correspondante.

15. Réacteur biosolaire selon la revendication 13 ou 14, **caractérisé en ce que** plusieurs éléments de réacteur (2) sont reliés par un élément de retenue (24) de manière à obtenir un panneau de réacteur (23).
